# EUROPEAN PATENT APPLICATION

(11) **EP 1 591 517 A1**
(43) Date of publication of application: **02.11.2005**
(21) Application number: 04405273.6
(22) Date of filing: 30.04.2004
(51) Int. Cl.: C12M 1/24

(54) **Lid for bioreactor bottles and/or centrifugation tubes**

(71) Applicant: Ismatec SA, Laboratoriumstechnik, 8152 Glattbrugg (CH)
(72) Inventor: Graf-Hausner, Ursula, 8405 Winterthur (CH); Kley, Martha, 8400 Winterthur (CH); Michels, Stephan, 8474 Dinhard (CH); Wetzel, Herbert, 8615 Wermatswil (CH); Müller, Uwe, 8006 Zürich (CH); Saxer, Daniel, 8048 Zürich (CH); Djojoatmodjo, Antono, 8625 Gossau (CH)
(74) Representative: Bremi, Tobias Hans

(57) **Abstract**

The present invention relates to a lid (2) for a substantially sterile bottle (1) in particular bioreactor bottle (1), comprising means (20) for securing the lid (2) to an upper opening of the bottle (1), a first pipe (3,4) for introducing media into the bottle (1), a second pipe (5,6,17) for removing media from the bottle (1), and a filter (11). It furthermore relates to a bioreactor system using such a lid (2) and it relates to a method of cultivating biomass in a bioreactor using such a lid (2). The system according to the invention allows highly automated and sterile cultivation of biomass using simple and reliable technology.

## Description

### TECHNICAL FIELD

The present invention relates to a lid for a substantially sterile bottle, in particular for a bioreactor bottle. It furthermore relates to a bioreactor system using such a lid and it relates to a method of cultivating biomass in a bioreactor using such a lid.

### BACKGROUND OF THE INVENTION

In the field of growing cell cultures on a lab scale, there is a need for increasing automation. Nowadays, the majority of steps involved in growing cell cultures for example for tissue regeneration but also generally in the field of handing liquids in this context is carried out manually which leads to high costs of such investigations or productions. It is however also prone to human errors and deviations due to inaccuracies and differences in handling between different samples or in repeated handling of the same sample which in the field of cell cultures may have substantial influence on the results obtained.

In the state-of-the-art, one can therefore find devices for at least partly automated growing of cells. For example GB 1539263 discloses an apparatus for growing cells which comprises a plurality of stacked dishes at least one of them or the whole setup being provided with the possibility of exchanging media, such that a partially automated growth under controlled conditions is possible in each of the dishes. A similar array of dishes is disclosed in FR 841569 describing a tower of dishes which can also at least partially be operated automatically.

One more recent disclosure is given in WO 93/10211 which also describes a cell cultivating device with separate, tray-like members or dishes which are handled within one bioreactor flask or bottle, i.e. which are located within one single sterile housing with one opening which can be closed by a cap.

### SUMMARY OF THE INVENTION

The objective problem underlying the present invention is therefore to provide means or tools for an increased automation in the field of growing cell cultures but also in the field of centrifugation of cell cultures and the like, and which in particular allows to reduce the number of human manipulations in the course of preparing, maintaining or controlling, and sampling of bioreactor containers like bottles, flasks, dishes, and the like.

The present invention solves the above problem by providing a particular lid for a substantially sterile bottle, i.e. for example a bioreactor bottle or flask, or for a centrifugation flask. This lid or cap comprises means for securing the lid to an opening, typically an upper or side opening of the bottle, such means for example being a clip mechanism or a threading which cooperate with corresponding devices provided on the bottle or flask or a neck thereof. The lid or cap is provided with a first pipe for normally introducing media into the bottle, i.e. with a first pipe which penetrates the lid. The lid or cap is additionally provided with a second pipe for normally removing media from the bottle, i.e. with a second pipe which also penetrates the lid. Furthermore, exchange of gas and/or maintenance of sterile conditions as well as maintenance of stable pressure conditions is made possible by providing a filter, or more general a gas exchange pathway, which allows the exchange of gas/air between the inside and the outside of the bottle in a sterile manner. The gas may well be air, but also the specific supply with other gases like carbon dioxide in a desired amount (e.g. 5%) for the stabilisation of the pH-value in the bottle is possible.

The object of the present invention is therefore a lid according to claim 1, a bioreactor system according to claim 12 and a method for cultivating biomass according to claim 13.

The key feature of the invention is therefore the fact that the lid according to the present invention allows to, in a surprisingly simple manner, introduce media like liquids into the bioreactor flask in a preferably sterile manner, but also to take out media like liquids, for example samples for monitoring the development within the bottle, also preferably in a sterile manner. While according to the state-of-the-art, such exchange of liquids involves manipulations of the bottle, like for example opening the cap and introducing or taking out liquids or generally media, this is not necessary any more here. Additionally, due to the fact that for introducing media and for taking out media two different pathways are provided in the cap, such a cap can comply with highest standards with respect to sterility but also with respect to purity of the samples and correspondingly with respect to isolation and reliability as well as reproducibility of the processes possible within a bioreactor closed with such a cap.

In a first preferred embodiment of the present invention, the first pipe comprises a pipe connection protruding towards the outside of the bottle. This pipe connection protruding towards the outside of the bottle allows easy but secure connection to piping directly, but it also allows to connect standard connection aids like for example standard Luer-Lock connections. Therefore, compatibility with pre-existing equipment in the lab is given.

Additionally or alternatively it is possible to have a pipe portion of the first pipe pointing into the bottle. In this case, preferentially the pipe portion of the first pipe pointing into the bottle has a length L such that media, in particular liquid media, can be introduced into the bottle or flask above the interface between a liquid (or solid) and a gaseous phase in the bottle. The terminal end of the pipe portion pointing into the bottle should preferably be at a position well above the liquid phase without wetting the inside of the bottle-neck 7, where there should be no cell growth. To allow this length to be adapted to different situations, it is, according to a preferred embodiment, also possible to provide an adjustable input pipe. This, in that for example the first pipe can be shifted within a sleeve or hole (with corresponding sealing means to make sure that sterile conditions within the bottle can be maintained) in the cap so that the position of the end of the pipe portion of the first pipe pointing into the bottle can be adapted by shifting the pipe in this sleeve to the appropriate position above the interface between a liquid (or solid) and a gaseous phase.

According to another preferred embodiment of the present invention, the second pipe comprises a pipe connection extending towards the outside of the bottle. Also here compatibility with existing lab equipment, like for example Luer-Lock connectors or the like, is thus given, and the pipe connection allows easy and safe connection to piping for removal of media from the bottle. Compatibility with standard sterile connectors allows changing the connectivity under sterile conditions. Additionally or alternatively a pipe portion of the second pipe can be provided pointing into the bottle. In this case, preferentially the pipe portion of the second pipe pointing into the bottle is connected to a tube extending substantially to the bottom of the bottle. Preferentially, this tube is a flexible tube. The tube preferably extends to one of the far bottom edges of the bottle with respect to the position of the lid. The tube may for example be made of PTFE or another material which is resistant to typical media used in such bioreactors. Also tubes coated with such resistant material are possible, like for example PTFE-coated tubing which can be at least partly flexible. The terminal end of the tube may have particular shapes apart from being cut orthogonal to the principal axis of the tube. For example a V-shaped end or a tilted cut end, but also an end provided with some kind of filter or barrier element to avoid intake of solid or dispersed material is possible depending on the field of use.

In order to allow adjustment of the terminal end of the tube connected to the second pipe within the bottle, and in order to correspondingly allow to position this terminal end at a location where removal of media is desired, the second pipe can be mounted rotatably within the lid in a corresponding sleeve or hole, wherein the rotation axis is substantially parallel to the principal axis of the pipe. Preferably, the terminal end of the tube connected to the second pipe within the bottle is thereby adjusted to point to a bottom corner of the far end of the bottle opposite to the lid. It is however also possible to adjust the terminal end of the tube to point into an upper corner of the far end of the bottle opposite to the lid, this for example to allow taking out of liquid only if the bottle is reversed upside down or put in an upright position. Since under normal conditions, the terminal end of the tube is immersed in the culture or at least in the liquid supernatant, the presence of the air/gas filter is important, since without this possibility of exchange and correspondingly of stable pressure conditions, input of media would automatically lead to an unwanted pushing out of media through the tube or to an undesired alteration of the growth conditions for the enclosed cell culture. Correspondingly, generally the air/gas filter is located in the lid as far up as possible and well separated from the input pipe to avoid contact with media and/or cell culture.

As already mentioned above, the lid should be equipped with means for fastening the lid to the neck of the bottle or flask. Preferentially to this end the lid is a screw lid, and this screw lid should be adapted to the bottle-neck of the bottle. To make sure that the positioning of the individual elements located within the lid (first and second pipe, filter) is well-defined if the lid is tightly connected to the bottle or flask, the screw lid should be adapted to the bottleneck of the bottle such that if the lid is secured to the bottle the functional elements of the lid are in a defined rotational position. To this end, it is possible to provide first indicator means on the lid and/or on the bottle for indicating the defined secured position of the lid. The first indicator means define the rotational position of the lid which corresponds to the internally provided dead stop position of the lid (abutment in the threading or the like). Correspondingly, the first indicator means indicate to the user, where the proper closed position is, which avoids unnecessary stress applied to sealing elements and the like. To allow easy handling, preferentially additionally second indicator means are provided on the lid for indicating the rotational position of the lid appropriate for putting the lid on to the bottle-neck, wherein there may additionally be provided signs for indicating the direction of rotation for closing the lid.

Another preferred embodiment of the present invention is characterised in that the bottle is a cell cultivating device comprising a container having a substantially flat bottom wall, side walls and a top wall and a closable filling opening being defined in one of said side walls. Said closable filling opening is carrying a bottleneck for the lid, wherein the axis of the substantially cylindrical bottleneck is preferentially upwardly tilted away from the plane of the bottom wall at an angle between 10°-45°. Preferentially, the length and the width of the cell cultivating device are substantially larger than its height. Typically, such a cell cultivating device has a length in the range of 5-20 centimetres, preferentially in the range of 8-15 centimetres, a width which is typically smaller than the length, the width being in the range of 5-15 centimetres, preferentially in the range of 7-10 centimetres. The height of the cell cultivating device on the other hand typically lies in the range of 1.5-6 centimetres, preferentially in the range of 2-5 centimetres.

In order to allow a simple and effective sterile connection with surrounding piping for input of media, it is advantageous to design the pipe connection of the first and/or the second pipe protruding towards the outside of the bottle cylindrically and with an end portion, the outer surface of which is conically converging towards the end, for example for cooperation with a female Luer-type connection element. Analogously, it may be advantageous to design the pipe connection of the first and/or the second pipe protruding towards the outside of the bottle cylindrically and with an end portion, the inner surface of which is cylindrical or conically opening towards the terminal outer end, preferably for cooperation with a male Luer-type connection element. In a most preferred embodiment, the terminal outer ends of the first and the second pipe have a different shape/geometry in order to avoid the connection of identical connectors and in order to only allow the connection of connectors with opposite polarity. Typically therefore, the outer terminal end of the input pipe is provided with a conical outer surface, while the outer terminal end of the output pipe is provided with a conical inner surface. Possible is for example the use of adapters of the type as known in the field of blood handling and blood collection. In this respect sterile adapters as available for the Monovette® system from Sarstedt (DE) under the order numbers 14.1205 or 14.1112 (multi adapters and membrane adapters) can be used. The use of such sterile connectors allow to amend the connectivity of the bottle under sterile conditions.

The present invention also relates to a bioreactor system comprising a cell cultivating device with a container having a substantially flat bottom wall, side walls and a top wall and a closable filling opening preferentially being defined in one of said side walls, said closable filling opening carrying a bottleneck for a lid, the axis of which substantially cylindrical bottleneck is preferentially upwardly tilted away from the plane of the bottom wall at an angle between 10°-45°, wherein preferentially the length and the width of the cell cultivating device are larger than its height, and a lid as given above.

Furthermore, the present invention relates to a method for cultivating biomass in a bioreactor, preferentially in a bioreactor system as detailed in the previous paragraph. The method is characterised in that for keeping liquid and solid media as well as the biomass, a bottle in the form of a cell cultivating device with a container having a substantially flat bottom wall, side walls and a top wall and a closable filling opening being defined in one of said side walls, said closable filling opening carrying a bottleneck for a lid, is used. The cell cultivating device is closed by means of a lid as described above and in particular liquid media are initially and/or periodically and/or finally introduced into the bottle using the first pipe, and media and/or samples are initially and/or periodically and/or finally taken from the bottle by means of the second pipe.

According to a first preferred embodiment of the method, the bottle is initially and/or finally and/or periodically and automatically moved mechanically, and either before, after and/or during this movement or between intervals of movement automatically media are introduced into the bottle. A fully automated growth and/or or sampling is therefore possible with the lid according to the present invention.

According to another preferred embodiment of the method, the bottle is initially and/or finally and/or periodically and automatically moved mechanically, and wherein either before, after and/or during this movement or between intervals of movement automatically media and/or samples are taken out of the bottle, preferentially for analytical purposes.

Further embodiments of the present invention, i.e. of the lid, of the bioreactor and/or of the method for cultivating biomass in a bioreactor are outlined in the dependent claims.

### SHORT DESCRIPTION OF THE FIGURES

In the accompanying drawings preferred embodiments of the invention are shown in which:
- Figure 1: shows in a) a central cut through a bioreactor flask, in b) an exploded perspective view of a bioreactor flask, in c) a view onto the lid, in d) and e) cuts as indicated by the respective arrows in figure 1c); and
- Figure 2: shows in a) a side view onto a centrifugation flask, in b) a view onto the cap of such a centrifugation flask, in c) an axial cut through a centrifugation flask according to the line A-A as indicated in figure 2a), and in d) an exploded perspective view of a centrifugation flask.

### DETAILED DESCRIPTION OF THE PREFERRED EMBODIMENTS

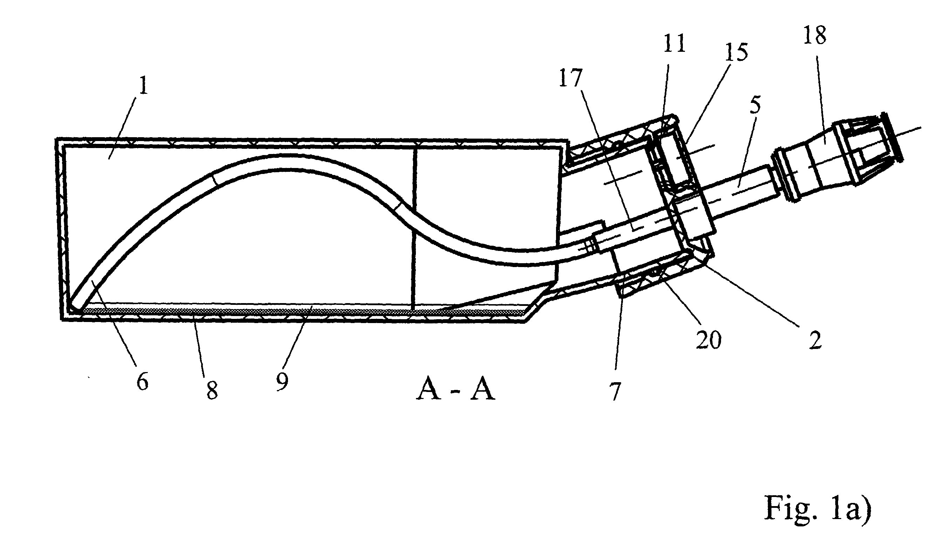

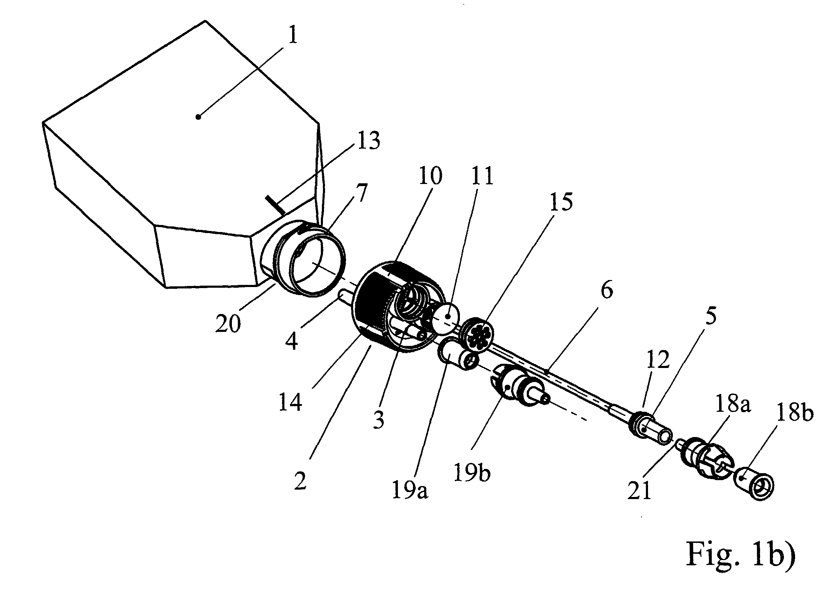

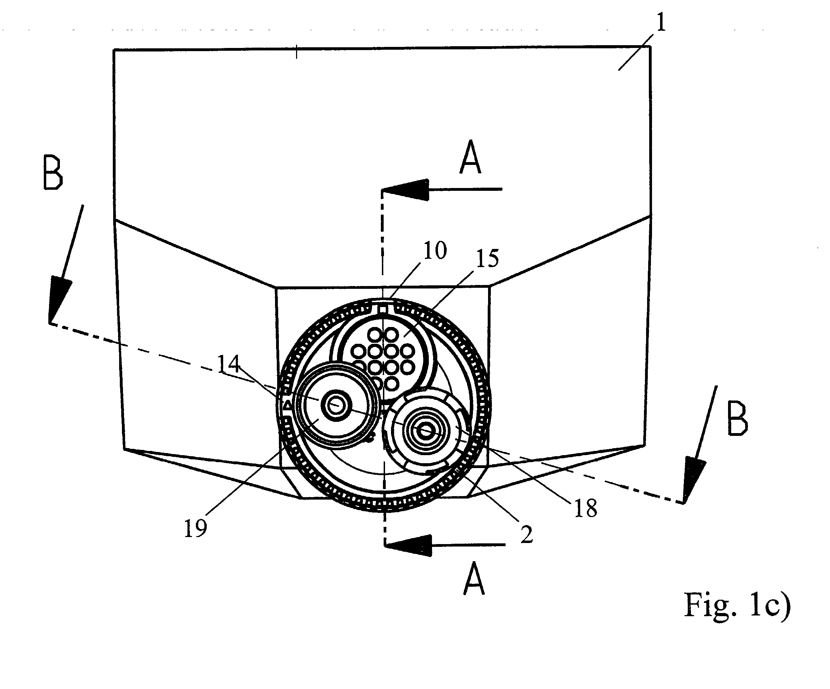

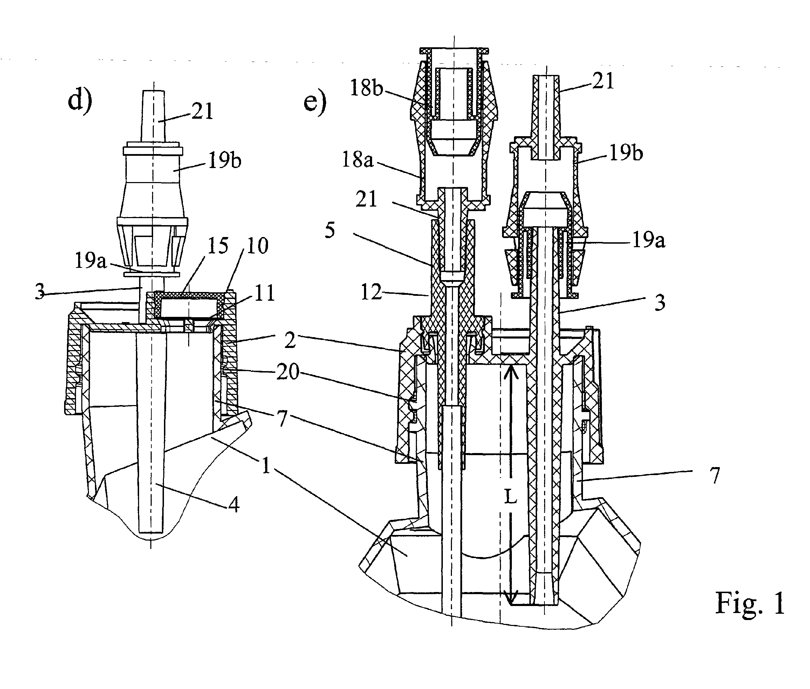

Referring to the drawings, which are for the purpose of illustrating the present preferred embodiments of the invention and not for the purpose of limiting the same, figure 1 shows a bioreactor bottle 1. Within the figures 1a) to 1e) like reference numerals designate like parts of the bioreactor bottle 1. The bottle 1 comprises a bottom wall, on which e.g. a biomass 8 is growing. The biomass 8 is covered by a liquid, or supernatant 9. The side walls of the bottle together with the bottom wall and the top wall enclose the actual reaction space which shall be kept sterile. The bottle comprises a bottleneck 7 which is provided on one side wall of forms integral part of thereof. The side wall which carries the bottleneck 7 usually comprises two converging portions joined by another portion which actually carries the bottleneck. This design proves to be advantageous for allowing the handling of liquids or generally media as comprehensively as possible by means of the opening of the bottleneck.

The bottleneck 7 is tilted upwardly with respect to the bottom wall of the bottle such that handling of media kept in the bottle is facilitated. The bottleneck can be closed by means of a cap 2 which is provided with an input pipe 3 which penetrates the lid 2. The input pipe has an outside portion 3 pointing towards the outside of the bottle and an inside portion 4, pointing into the bottle. On the outside portion 3, which has the form of a pipe connection, sterile standard connectors 19 like for example Luer-type connectors can be connected. Since in this case the input pipe should be used for introducing media, the outside portion 3 is provided with a conical outer surface, such that a female Luer-type connector 19a can be shifted onto the outside portion 3 and fastened to it by means of friction. To be able to use standard connectors 19 in the lab field, a top end outer diameter of approximately 3.5 millimetres expanding towards the cap to 4.5 millimetres is appropriate to cooperate with a standard female connector with a cylindrical or conically converging inner diameter of approximately 4 millimetres at its entrance.

Additionally, the lid 2 is provided with an output pipe, the outside portion of which is designated by reference numeral 5. The inside portion 17 of this output pipe is rather short and serves to allow the fixing of an output tube 6 to this pipe connection 17. The output tube 6, for example made of PTFE, has an outer diameter of approximately 3 millimetres and an inner diameter of approximately 2.5 millimetres. The length of this output tube 6 is adjusted such as to be slightly longer than the length of the bottle such that if the cap is fastened to the bottleneck, the output tube 6 is forced to bend and to extend to one of the far end corners of the bottle 1 and is automatically fixed in this position.

the outside portion 5 of the output pipe is designed such as to take up standard male laboratory type connectors 18. Since for example Luer-type male connectors may have a conical outer surface which at the very end has a diameter of approximately 3.5 millimetres expanding to approximately 4.5 millimetres, the outside portion 5 is designed to have a cylindrical or conically converging inner surface with a diameter at the entrance of approximately 4 millimetres to allow tight friction connection of such a standard connector 18 to the outside portion 5.

Since the positioning of the individual elements located within the lid 2 relative to the bottle is important for proper functioning, means are provided for defining a proper position of the lid 2. On the one hand, there is provided a position indicator 10 on the lid 2, which gives the closed position (indicated in a pictogram by the square as visible in Fig. 1c), if aligned with a corresponding indicator 13 on the bottle 1. On the other hand there is provided another position indicator 14, which shall be aligned with the indicator 13 if the cap is to be put onto the bottleneck 7 (here the direction of rotation is indicated in a pictogram by the triangle as visible in Fig. 1c). If the position indicator 14 is aligned with the indicator 13, a rotation of the lid 2 by 270° tightly fixes the cap to the bottle since there is a corresponding threading provided on the bottleneck 7 as well as on the inside of the cap (see below).

As one can see from figure 1a) which gives a central longitudinal cut, the cap not only comprises an input pipe and an output pipe but also a filter element, i.e. there is 3 bushings going through the cap 2, one for the input pipe, one for the output pipe, and one for the filter 11. The filter 11 is there to allow exchange of gas/air between the inside of the bottle and the outside of the bottle. This is necessary because the introduction or removal of media to or from the inside of the bottle would otherwise lead to differences in pressure inside the bottle, and because the diffusion of gas or air (in particular oxygen and/or nitrogen and/or carbon dioxide) into the inside of the bottle is often necessary for the processes within the bottle.

It has to be pointed out that in principle also the filter may be equipped with a pipe connection towards the outside of the bottle if specific gases or mixtures of gases have to be supplied to the bottle.

In order to make sure that sterile conditions can be maintained, the filter comprises the actual filter material or filter membrane 11 which is kept in a specific position in a bore in the cap by means of a cover plate 15. The cover plate 15 is provided with holes such that exchange of air as possible. Such a filter may for example be based on PTFE on a support material. The actual filter membrane 11 is pressed to the bottom (abutment provided in the bore in the lid) by means of the cylindrical walls of the cover plate 15. It is important that the filter membrane 11 is pressed down and held to cover the full cross-section of the bore in the lid, since if e.g. the filter material is only provided in the hollow space enclosed by the cylindrical walls of the cover plate 15, unwanted germs or the like may creep through the gap between the cylindrical wall and the wall of the bore in the lid that takes up the filter assembly. As one can also see in figure 1a) and d) the cap is provided with a thread 20, and so is the bottleneck 7 with a corresponding thread 20.

Figure 1b), giving an exploded view of the bottle 1 with the cap 2 clearly indicates the individual parts. In this specific example, the connectors 18 and 19 are so-called Luer-lock connectors which are used with opposite polarity for the input pipe and for the output pipe.

Also visible in figure 1b) is the feature that the output pipe is designed as a rotatable insert 12 which is located in a corresponding bushing or sleeve or hole within the lid 2. This rotatable insert 12 towards the inside carries the inside portion 17 and towards the outside the outside portion 5. There is a slight interference fit between the insert 12 and its seat in the lid 2 to ensure sealing. The fact that the output pipe is designed rotatably can be used for adjusting the position of the output tube 6 within the bottle 1. If the lid is screwed onto the neck of the bottle 11, it may well be that the output pipe 6 points to an upward corner of the bottle instead of the desired lowermost corner of the bottle. If this happens, one can, by means of rotating the insert 12 for example by holding it on the outside portion 5, rotate the insert 12 and correspondingly the position of the output tube 6.

Figure 1c) gives a view onto the lid 2, and it indicates the relative arrangement of the input pipe, the output pipe, and the filter. Indicated in figure 1c) are in particular the cuts as displayed in figures 1d) and e).

In figure 1d) it can be seen that the cover plate 15 comprises substantially cylindrical sidewalls which cooperate with the corresponding sidewalls of the hole of the lid 2, wherein a tight fixing of the cover plate 15 within the lid is provided by means of a key and slot structure. The circular edge of the sidewalls press the filter membrane 11 against an abutment or recess of the bore.

In Fig. 1e) one can additionally see the key and slot structure which holds the insert in the corresponding bore in the lid, which, while still allowing to turn the insert keeps sterile conditions, i.e. seals the inside of the bottle from the outside.

It has to be pointed out that a bottle or flask according to figure 1 can be used in automated biomass handling. Once the biomass is introduced into the bottle under sterile conditions, the cap 2 can be screwed onto the neck of the bottle, and, if need be, the position of the output tube 6 can be adjusted by means of rotation of the rotatable insert 12. Subsequently, the standard sterile connectors 18a as well as 19a, if not yet provided, can be connected to the pipe connections 3 and 5, respectively (still under sterile conditions, which is subsequently not necessary anymore, since the inside of the bottle is then isolated). In the present example adapters of the type as known in the field of blood handling and blood collection are used. Particularly, sterile adapters as available for the Monovette® system from Sarstedt (DE) under the order numbers 14.1205 or 14.1112 (multi adapters and membrane adapters) are given in the figures. Subsequently, piping allowing the transport of media to be inserted into the bottle and provided with a standard male terminal connection 19b can be connected to the connector 19a. Analogously, piping allowing the transport of media out of the bottle provided with a standard female connector 18b can be connected to the connector 18a.

In principle, it is possible to also take out gas via the input pipe 4 and two input media via the tube 6. In the preferred mode of operation however, media, be it solid, liquid or gas, are introduced via tube 4 and media are taken by means of tube 6. Normally, the terminal end of tube 6 is fully immersed in at least the liquid phase present in the bottle. The present setup by providing a tube 4 the end of which under normal handling conditions is located well above the liquid/gas phase present in the bottle for input of media automatically and effectively provides a sterile barrier.

During automated processing, which may involve specific movement of the bottle, temperature profiles, and the like, liquids can now be introduced and taken out without having to open the bottle. Additionally, if different liquids have to be introduced into the bottle, or if media taken out of the bottle have to be transported along a different pathway, connecting a different piping under sterile conditions is easily possible since the sterile connectors 18 and 19 allow a fast and sterile amendment of the connectivity.

Figure 1e) indicates the structure of the inside portion 4 in more detail, and it specifically indicates its length L, which is generally adapted such as to allow introduction of media at a position well above the liquid phase without wetting the inside of the bottle-neck 7 where there should be no cell growth.

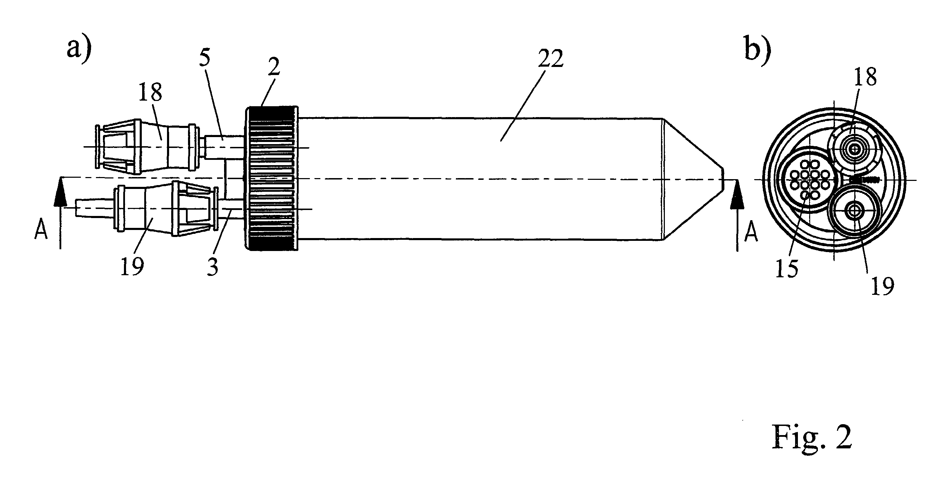

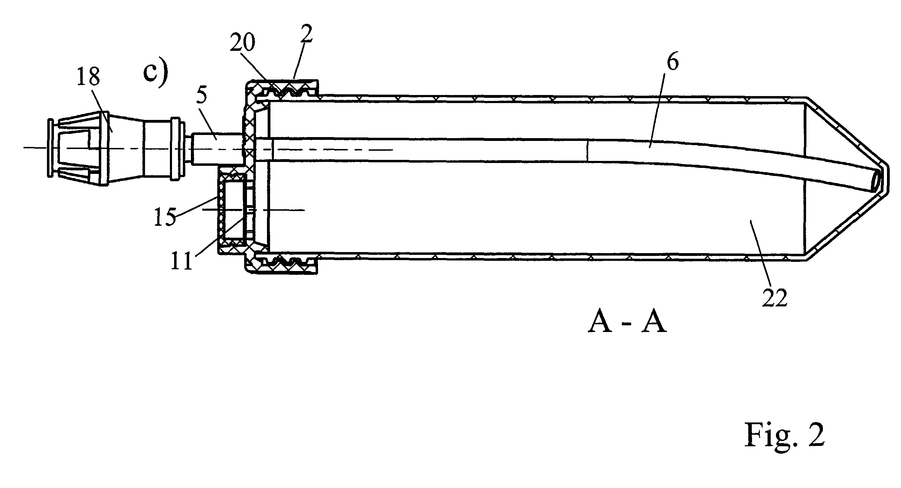

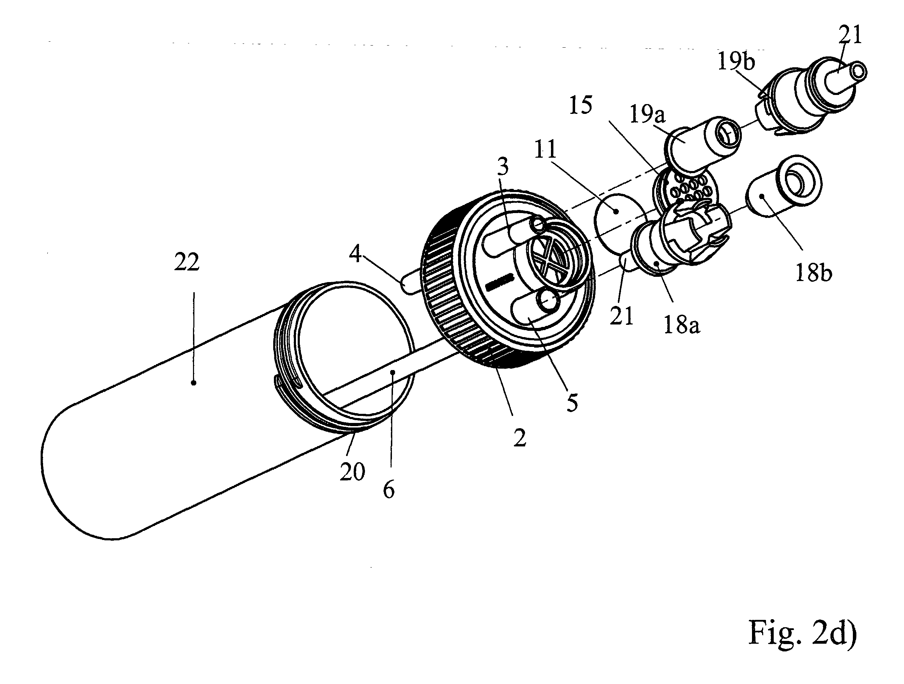

Figure 2 gives an alternative example, namely a centrifugation flask equipped with an equivalent lid 2. Again the lid 2 comprises a filter element 11, an input pipe and an output pipe, the three of them being arranged almost symmetrically in the lid. As one can see from figure 2c), the output tube 6 has a length such as to extend to the very bottom of the centrifugation tube 22, which is achieved by giving the output tube 6 a length which is slightly larger than the height of the centrifugation tube 22 such that the output tube 6 is slightly bent and forced into the very bottom of the centrifugation tube 22. The output tube 6 may also have a terminal opening with a tilted cut end.

### LIST OF REFERENCE NUMERALS

- 1: bottle, bioreactor, flask
- 2: lid, cover, cap
- 3: input pipe, outside portion
- 4: input pipe, inside portion
- 5: output pipe, outside portion
- 6: output tube
- 7: bottle-neck
- 8: bottom of 1, biomass
- 9: supernatant
- 10: position indicator (final position)
- 11: filter, filter membrane
- 12: rotatable insert
- 13: position indicator on bottle
- 14: position indicator (initial position)
- 15: cover plate
- 17: output pipe, inside portion
- 18: standard sterile connector (Luer-type, Luer-lock) for input pipe
- 19: standard sterile connector (Luer-type, Luer-lock) for output pipe
- 20: thread
- 21: pipe connection
- 22: centrifugation flask

## Claims

1. Lid (2) for a substantially sterile bottle (1) in particular bioreactor bottle (1), comprising
means (20) for securing the lid (2) to a preferentially upper or side opening of the bottle (1)
a first pipe (3,4) for introducing media into the bottle (1),
a second pipe (5,6,17) for removing media from the bottle (1),
and a filter (11) allowing the exchange of gas or air between the outside and the inside of the bottle (1).

2. Lid (2) according to claim 1, wherein the first pipe (3,4) comprises a pipe connection (3) protruding towards the outside of the bottle (1) and/or a pipe portion (4) pointing into the bottle (1).

3. Lid (2) according to claim 2, wherein the pipe portion (4) of the first pipe pointing into the bottle (1) has a length (L) such that media, in particular liquid media, can be introduced into the bottle (1) substantially above the interface between a liquid and a gaseous phase in the bottle (1), wherein preferably means are provided allowing to adjust the length (L) of the pipe portion (4).

4. Lid (2) according to any of the preceding claims, wherein the second pipe (5,6,17) comprises a pipe connection (5) protruding towards the outside of the bottle (1) and/or a pipe portion (17) pointing into the bottle (1).

5. Lid (2) according to claim 4, wherein the pipe portion (17) of the second pipe pointing into the bottle (1) is connected to a tube (6) extending to the bottom (8) of the bottle (1), preferentially to one of the far edges of the bottom (8) with respect to the position of the lid (2), wherein preferably said to tube is made of PTFE.

6. Lid (2) according to claim 5, wherein the second pipe (5,6,17) is rotatably mounted within the lid (2) wherein the rotation axis is substantially parallel to the principal axis of the pipe.

7. Lid (2) according to any of the preceding claims, wherein it is a screw lid, and wherein preferentially the screw lid (2) is adapted to the bottle-neck (7) of the bottle (1) such that if the lid (2) is secured to the bottle (1) the functional elements (3,4,5,6,11,17) of the lid (2) are in a defined rotational position.

8. Lid (2) according to claim 7, wherein first indicator means (10,13) are provided on the lid (2) and/or on the bottle (1) for indicating the secured position of the lid (2), and wherein preferentially additionally second indicator means (14) are provided on the lid (2) for indicating the rotational position of the lid (2) appropriate for putting the lid (2) on to the bottle-neck (7).

9. Lid (2) according to any of the preceding claims, wherein the bottle is a cell cultivating device comprising a container having a substantially flat bottom wall, side walls and a top wall and a closable filling opening being defined in one of said side walls, said closable filling opening carrying a bottleneck (7) for the lid (2), the axis of which substantially cylindrical bottleneck (7) is preferentially upwards tilted away from the plane of the bottom wall at an angle between 10°-45°, wherein preferentially the length and the width of the cell cultivating device are substantially larger than its height.

10. Lid (2) according to any of the preceding claims, wherein the pipe connection (3,5) of the first and/or the second pipe protruding towards the outside of the bottle (1) is cylindrical and has an end portion the outer surface of which is conically converging towards the end, preferably for cooperation with a female Luer-type connection.

11. Lid (2) according to any of the preceding claims, wherein the pipe connection (3,5) of the first and/or the second pipe protruding towards the outside of the bottle (1) is cylindrical and has an end portion the inner surface of which is conically opening towards the end, preferably for cooperation with a male Luer-type connection.

12. Bioreactor system comprising a cell cultivating device with a container having a substantially flat bottom wall, side walls and a top wall and a closable filling opening being defined in one of said side walls, said closable filling opening carrying a bottleneck (7) for a lid (2), the axis of which substantially cylindrical bottleneck (7) is preferentially upwards tilted away from the plane of the bottom wall at an angle between 10°-45°, wherein preferentially the length and the width of the cell cultivating device are substantially larger than its height, and a lid (2) according to any of the preceding claims.

13. Method for cultivating biomass in a bioreactor, preferentially in a bioreactor system according to claim 12,
wherein a bottle in the form of a cell cultivating device (1) with a container having a substantially flat bottom wall, side walls and a top wall and a closable filling opening being defined in one of said side walls, said closable filling opening carrying a bottleneck (7) for a lid (2), is used for keeping liquid and solid media as well as the biomass,
wherein the cell cultivating device is closed by means of a lid (2) according to any of the claims 1-11,
wherein in particular liquid media are initially and/or periodically introduced into the bottle (1) using the first pipe (3,4), and wherein media and/or samples are taken from the bottle by means of the second pipe (5,6,17).

14. Method according to claim 13, wherein the bottle is initially, finally and/or periodically and automatically moved mechanically, and wherein either during this movement or between intervals of movement automatically media are added into the bottle.

15. Method according to claim 13 or 14, wherein the bottle is initially, finally and/or periodically and automatically moved mechanically, and wherein either during this movement or between intervals of movement automatically media and/or samples are taken from the bottle, preferentially for analytical purposes.
